# EUROPEAN PATENT APPLICATION

(11) **EP 4 162 971 A1**
(43) Date of publication of application: **12.04.2023**
(21) Application number: 21823063.9
(22) Date of filing: 08.06.2021
(51) Int. Cl.: A61M 60/113, A61M 60/268, A61M 1/26, A61M 1/36

(54) **BLOOD PUMP FOR CAUSING BLOOD TO FLOW IN ONE DIRECTION AND BLOOD OXIDATION SYSTEM COMPRISING SAME**

(30) Priority: 09.06.2020 KR 20200069530
(71) Applicant: Samsung Life Public Welfare Foundation, Seoul 04348 (KR)
(72) Inventor: CHO, Yang Hyun, Seoul 06503 (KR); CHOI, Seong Wook, Chuncheon-si Gangwon-do 24415 (KR)
(74) Representative: Heubeck, Christian
(86) International application number: PCT/KR2021/007178
(87) International publication number: WO 2021/251737

(57) **Abstract**

The present invention relates to a blood pump for causing blood to flow in one direction and a blood oxidation system comprising same and, more particularly, to an oxidation system for circulating blood in a body to an oxidizer so as to supply oxygen into the body from outside the body and a blood pump used for the oxidation system to cause blood to flow in one direction. The blood pump according to an embodiment of the present invention may comprise: an outer wall having a gas outlet port formed through a lateral surface thereof; a blood chamber having a pouch which is inserted into the outer wall and of which opposite ends are connected to opposite ends of the outer wall, respectively; a pump injection valve fluid-communicably coupled to one end of the blood chamber and blocking blood from flowing to the blood chamber; and a pump discharge valve fluid-communicably coupled to the other end of the blood chamber and blocking blood from flowing from the blood chamber.

## Description

### Technical Field

The present disclosure relates to a blood pump for forcing blood to flow in one direction and an oxygenation system including the same, and more particularly, to an oxygenation system for circulating blood of the body to an oxygenator so that oxygen is supplied from the outside of the body to the inside of the body, and a blood pump that is used in the oxygenation system and forces blood to flow in one direction.

### Background Art

Extracorporeal membrane oxygenation (ECMO) which supplies oxygen to the body by circulating blood to the outside of the body is used in a patient whose pulmonary function does not operate normally, etc.

Such an extracorporeal blood oxygenation system includes a blood pump that forces blood to flow in one direction so that blood is circulated between the outside of the body and the inside of the body.

The blood pump is divided into a pulsatile blood pump and a non-pulsatile blood pump depending on a method of a blood stream occurring. The non-pulsatile blood pump has a function for enabling blood to be pushed out by rotatory power through rotating an impeller that directly comes into contact with blood at high speed. The pulsatile blood pump has a function for enabling blood therein to be ejected by pressing a blood pouch having an artificial ventricle function or a portion in which blood is contained on the outside thereof.

An apparatus that causes a pressure change in the blood pouch of the pulsatile blood pump is divided into a mechanical type apparatus for applying pressure by pressing the mechanical type apparatus by using a tool having a solid form and a pneumatic type apparatus for applying pressure by using pressurized gas. In the pneumatic pulsatile blood pump, compressed air supplied from the outside compresses the blood pouch. Pressure of the blood pouch is restored into negative pressure by a vacuum pump. Check valves, such as valves, are installed at the blood inflow port and outflow port of the blood pouch so that blood is transferred in one direction by the contraction and restoration of the blood pouch.

The non-pulsatile blood pump corresponding to a centrifugal pump has problems in that damage to a blood corpuscle occurs due to high shear stress caused by highspeed rotation, a motor and a controller have great volumes and masses in order to supply a sufficient output, and the transfer of the non-pulsatile blood pump is difficult.

Furthermore, the pulsatile blood pump has problems in that it may require a blood transfusion because the volume of blood stored in the blood pouch, etc. is large and a risk of crack damage to the blood pouch is high because an excessive force acts on the blood pouch when the blood pouch is contracted or expanded. Furthermore, the pulsatile blood pump has problems in that the durability of a valve is low and the blood pump needs to be frequently repaired or replaced because a blood clot is generated due to the stagnation of blood in a check valve, that is, a component provided in order to prevent the backflow of blood.

### DISCLOSURE

### Technical Problem

In order to solve the aforementioned problems, an object of the present disclosure is to provide an extracorporeal blood oxygenation system having low power consumption, a small size, and a light weight by using gas pressure energy of a high-pressure oxygen tank in the circulation of a blood flow.

Furthermore, an object of the present disclosure is to provide a pulsatile blood pump for transferring blood in one direction even without a check valve and an extracorporeal blood oxygenation system including such a blood pump.

Furthermore, an object of the present disclosure is to provide an extracorporeal blood oxygenation system which enables control of the heart and the counterpulsation, solves distension of the ventricles, lowers the burden of the heart, improves coronary circulation, and has the improved supply of blood and gas inside the body.

### Technical Solution

As an embodiment of the present disclosure, a blood pump that forces blood to flow in one direction is provided.

A blood pump according to an embodiment of the present disclosure may include a blood chamber including an outer wall configured to have a gas inflow/outflow port formed at one end thereof and a pouch inserted into the outer wall and configured to have both ends coupled to both ends of the outer wall, respectively, an inlet valve for a pump coupled to one end of the blood chamber in a way that a fluid communicates therewith and configured to block a flow of blood to the blood chamber, and an outlet valve for a pump coupled to the other end of the blood chamber in a way that a fluid communicates therewith and configured to block a flow of blood from the blood chamber.

In the blood pump according to an embodiment of the present disclosure, in the blood chamber, pneumatic pressure may be formed between the outer wall and the pouch by a gas that is introduced and discharged through the gas inflow/outflow port.

In the blood pump according to an embodiment of the present disclosure, in the blood chamber, an outward form of each of the outer wall and the pouch may be a bent pipe shape.

In the blood pump according to an embodiment of the present disclosure, one side of the pouch may be deformed by pneumatic pressure so that a flow of blood is formed.

In the blood pump according to an embodiment of the present disclosure, the pouch may be formed in a hollow form having an elastic restoring force.

In the blood pump according to an embodiment of the present disclosure, the inlet valve for a pump or the outlet valve for a pump may further include a valve outer wall having the gas inflow/outflow port formed at one end thereof, and a valve pouch inserted into the valve outer wall and configured to have both ends coupled to both ends of the valve outer wall, respectively.

In the blood pump according to an embodiment of the present disclosure, an outward form of each of the valve outer wall and the valve pouch may be a bent pipe shape.

In the blood pump according to an embodiment of the present disclosure, one side of the valve pouch may be deformed by pneumatic pressure so that a flow of blood is blocked.

In the blood pump according to an embodiment of the present disclosure, the valve pouch may be formed in a hollow form having an elastic restoring force.

The blood pump according to an embodiment of the present disclosure may further include a connection unit configured to have both ends coupled to the blood chamber and the inlet valve for a pump or the outlet valve for a pump, respectively, and to have an internal diameter that is increased from an end of the connection unit coupled to the inlet valve for a pump or the outlet valve for a pump toward an end of the connection unit coupled to the blood chamber.

As an embodiment of the present disclosure, a blood oxygenation system is provided.

A blood oxygenation system according to an embodiment of the present disclosure may include the aforementioned blood pump, a pressure tank configured to accommodate a compressed gas, an oxygenator configured to oxidize blood that is circulated by the blood pump, a connection pipe that is a moving passage of the gas and configured to connect the blood pump and the pressure tank or the oxygenator, a plurality of valves disposed in the connection pipe and configured to control a flow of the gas that is introduced and discharged through the blood pump, and a controller configured to control an operation of the blood pump by opening and closing the valves.

The blood oxygenation system according to an embodiment of the present disclosure may include the plurality of blood pumps. The controller may control the plurality of blood pumps so that blood is discharged from some of the plurality of blood pumps and blood is introduced into the remaining blood pumps of the plurality of blood pumps.

The blood oxygenation system according to an embodiment of the present disclosure may further include a pneumatic pressure pump disposed in a connection pipe that connects the blood pump and the oxygenator and configured to form vacuum in the blood pump by sucking the gas supplied to the blood pump.

In the blood oxygenation system according to an embodiment of the present disclosure, the gas may be oxygen, and the gas may be moved from the pressure tank to the blood pump and from the blood pump to the oxygenator through the connection pipe.

The blood oxygenation system according to an embodiment of the present disclosure may further include a plurality of pressure sensors disposed in the blood chamber, the inlet valve for a pump, and the outlet valve for a pump, respectively.

In the blood oxygenation system according to an embodiment of the present disclosure, the controller may determine whether the valve has failed based on pressure measured by the pressure sensor in a preset condition.

In the blood oxygenation system according to an embodiment of the present disclosure, the valves may include a connection valve including a first connection valve having one end connected to the inlet valve for a pump, a second connection valve having one end connected to the blood chamber, and a third connection valve having one end connected to the outlet valve for a pump, an inlet valve including a first inlet valve having both ends connected to the pressure tank and the first connection valve, respectively, a second inlet valve having both ends connected to the pressure tank and the second connection valve, respectively, and a third inlet valve having both ends connected to the pressure tank and the third connection valve, respectively, and an outlet valve including a first outlet valve having both ends connected to the oxygenator and the first connection valve, respectively, a second outlet valve having both ends connected to the oxygenator and the second connection valve, respectively, and a third outlet valve having both ends connected to the oxygenator and the third connection valve, respectively.

In the blood oxygenation system according to an embodiment of the present disclosure, the controller may enable the blood chamber to perform a series of operations. The operation may include supplying the gas into the inlet valve for a pump by opening the first inlet valve in a state in which only the third outlet valve has been opened, supplying the gas into the blood chamber by opening the second inlet valve and closing the third outlet valve, supplying the gas into the outlet valve for a pump by closing the first inlet valve and opening the third inlet valve, sucking the gas from the inlet valve for a pump by closing the second inlet valve and opening the first outlet valve, sucking the gas from the blood chamber by closing the third inlet valve and opening the second outlet valve, and sucking the gas from the outlet valve for a pump by closing the first outlet valve and opening the third outlet valve.

In the blood oxygenation system according to an embodiment of the present disclosure, the controller may enable the blood chamber to repeatedly perform the operation. The number of repetitions may be calculated on the basis of a heart rate of a patient.

### Advantageous Effects

According to the present disclosure, the generation of a blood clot can be prevented by providing the inlet and outlet valves for a pump, which are operated by pneumatic pressure in order to block a flow of blood, at both ends of the blood chamber that generates a flow of blood within the blood pump so that blood is prevented from coming into contact with metal.

Furthermore, according to the present disclosure, the portability of the oxygenation system can be improved by replacing an actuator having a large volume and weight by using high-pressure oxygen gas as a driving source for a blood stream.

Effects which may be obtained in the present disclosure are not limited to the aforementioned effects, and other effects not described above may be evidently understood by a person having ordinary knowledge in the art to which the present disclosure pertains from the following description.

### Brief Description of Drawings

FIG. 1 is a cross-sectional view of the entire construction of a blood pump according to an embodiment of the present disclosure.
FIG. 2 is a diagram illustrating a structure and connection relation of components of the blood pump according to an embodiment of the present disclosure.
FIG. 3 is a diagram illustrating an operating process of the blood pump according to an embodiment of the present disclosure.
FIG. 4 is a diagram illustrating an operation of the blood pump depending on a location of a gas inflow/outflow port of the blood pump according to an embodiment of the present disclosure.
FIG. 5 illustrates various embodiments of the blood pump according to the present disclosure, which are differently implemented depending on their purposes.
FIG. 6 is a block diagram of a blood oxygenation system according to an embodiment of the present disclosure.
FIG. 7 is a graph illustrating the amounts of blood that is ejected by the blood pump before and after the component of a pneumatic pressure pump is added in the blood oxygenation system according to an embodiment of the present disclosure.
FIGS. 8 to 13 are diagrams illustrating an operation that is performed by a controller of the blood oxygenation system according to an embodiment of the present disclosure.

### Best Mode

As an embodiment of the present disclosure, a blood pump that forces blood to flow in one direction is provided.

A blood pump according to an embodiment of the present disclosure may include a blood chamber including an outer wall configured to have a gas inflow/outflow port formed at one end thereof and a pouch inserted into the outer wall and configured to have both ends coupled to both ends of the outer wall, respectively, an inlet valve for a pump coupled to one end of the blood chamber in a way that a fluid communicates therewith and configured to block a flow of blood to the blood chamber, and an outlet valve for a pump coupled to the other end of the blood chamber in a way that a fluid communicates therewith and configured to block a flow of blood from the blood chamber.

In the blood pump according to an embodiment of the present disclosure, in the blood chamber, pneumatic pressure may be formed between the outer wall and the pouch by a gas that is introduced and discharged through the gas inflow/outflow port.

In the blood pump according to an embodiment of the present disclosure, in the blood chamber, an outward form of each of the outer wall and the pouch may be a bent pipe shape.

In the blood pump according to an embodiment of the present disclosure, one side of the pouch may be deformed by pneumatic pressure so that a flow of blood is formed.

In the blood pump according to an embodiment of the present disclosure, the pouch may be formed in a hollow form having an elastic restoring force.

In the blood pump according to an embodiment of the present disclosure, the inlet valve for a pump or the outlet valve for a pump may further include a valve outer wall having the gas inflow/outflow port formed at one end thereof, and a valve pouch inserted into the valve outer wall and configured to have both ends coupled to both ends of the valve outer wall, respectively.

In the blood pump according to an embodiment of the present disclosure, an outward form of each of the valve outer wall and the valve pouch may be a bent pipe shape.

In the blood pump according to an embodiment of the present disclosure, one side of the valve pouch may be deformed by pneumatic pressure so that a flow of blood is blocked.

In the blood pump according to an embodiment of the present disclosure, the valve pouch may be formed in a hollow form having an elastic restoring force.

The blood pump according to an embodiment of the present disclosure may further include a connection unit configured to have both ends coupled to the blood chamber and the inlet valve for a pump or the outlet valve for a pump, respectively, and to have an internal diameter that is increased from an end of the connection unit coupled to the inlet valve for a pump or the outlet valve for a pump toward an end of the connection unit coupled to the blood chamber.

As an embodiment of the present disclosure, a blood oxygenation system is provided.

A blood oxygenation system according to an embodiment of the present disclosure may include the aforementioned blood pump, a pressure tank configured to accommodate a compressed gas, an oxygenator configured to oxidize blood that is circulated by the blood pump, a connection pipe that is a moving passage of the gas and configured to connect the blood pump and the pressure tank or the oxygenator, a plurality of valves disposed in the connection pipe and configured to control a flow of the gas that is introduced and discharged through the blood pump, and a controller configured to control an operation of the blood pump by opening and closing the valves.

The blood oxygenation system according to an embodiment of the present disclosure may include the plurality of blood pumps. The controller may control the plurality of blood pumps so that blood is discharged from some of the plurality of blood pumps and blood is introduced into remaining blood pumps of the plurality of blood pumps.

The blood oxygenation system according to an embodiment of the present disclosure may further include a pneumatic pressure pump disposed in a connection pipe that connects the blood pump and the oxygenator and configured to form vacuum in the blood pump by sucking the gas supplied to the blood pump.

In the blood oxygenation system according to an embodiment of the present disclosure, the gas may be oxygen, and the gas may be moved from the pressure tank to the blood pump and from the blood pump to the oxygenator through the connection pipe.

The blood oxygenation system according to an embodiment of the present disclosure may further include a plurality of pressure sensors disposed in the blood chamber, the inlet valve for a pump, and the outlet valve for a pump, respectively.

In the blood oxygenation system according to an embodiment of the present disclosure, the controller may determine whether the valve has failed based on pressure measured by the pressure sensor in a preset condition.

In the blood oxygenation system according to an embodiment of the present disclosure, the valves may include a connection valve including a first connection valve having one end connected to the inlet valve for a pump, a second connection valve having one end connected to the blood chamber, and a third connection valve having one end connected to the outlet valve for a pump, an inlet valve including a first inlet valve having both ends connected to the pressure tank and the first connection valve, respectively, a second inlet valve having both ends connected to the pressure tank and the second connection valve, respectively, and a third inlet valve having both ends connected to the pressure tank and the third connection valve, respectively and an outlet valve including a first outlet valve having both ends connected to the oxygenator and the first connection valve, respectively, a second outlet valve having both ends connected to the oxygenator and the second connection valve, respectively, and a third outlet valve having both ends connected to the oxygenator and the third connection valve, respectively.

In the blood oxygenation system according to an embodiment of the present disclosure, the controller may enable the blood chamber to perform a series of operations. The operation may include supplying the gas into the inlet valve for a pump by opening the first inlet valve in a state in which only the third outlet valve has been opened, supplying the gas into the blood chamber by opening the second inlet valve and closing the third outlet valve, supplying the gas into the outlet valve for a pump by closing the first inlet valve and opening the third inlet valve, sucking the gas from the inlet valve for a pump by closing the second inlet valve and opening the first outlet valve, sucking the gas from the blood chamber by closing the third inlet valve and opening the second outlet valve, and sucking the gas from the outlet valve for a pump by closing the first outlet valve and opening the third outlet valve.

In the blood oxygenation system according to an embodiment of the present disclosure, the controller may enable the blood chamber to repeatedly perform the operation. The number of repetitions may be calculated on the basis of a heart rate of a patient.

### Mode for Invention

Hereinafter, embodiments of the present disclosure are described in detail with reference to the accompanying drawings so that a person having ordinary knowledge in the art to which the present disclosure pertains may easily practice the embodiments. However, the present disclosure may be implemented in various different forms and is not limited to the embodiments described herein. Furthermore, in the drawings, in order to clearly describe the present disclosure, a part not related to the description is omitted, and a similar reference number is used to refer to a similar part throughout the specification.

Terms used in this specification are briefly described, and the present disclosure is described in detail.

Terms used in the present disclosure are common terms currently widely used and selected by taking into consideration functions in the present disclosure, but the terms may be changed depending on an intention of a technician skilled in the art, a precedent, or the advent of a new technology. Furthermore, in a specific case, some terms are randomly selected by the applicant. In this case, the meaning of a corresponding term will be described in detail in the corresponding description of the invention. Accordingly, terms used in the present disclosure should be defined based on their meanings and contents over the present disclosure, not the simple names of the terms.

Throughout the specification, unless explicitly described to the contrary, when any part "includes" another element, it indicates the further inclusion of other elements, not the exclusion of other elements. Furthermore, the term "... unit" or "module" described in the specification means a unit for processing at least one function or operation, and the unit or module may be implemented by hardware or software or a combination of hardware and software. Furthermore, throughout the specification, when it is described that one part is "connected" to another part, the one part may be "directly connected" to the another part or may be connected to the another part "with a still another part interposed therebetween."

The present disclosure is described in detail below with reference to the accompanying drawings.

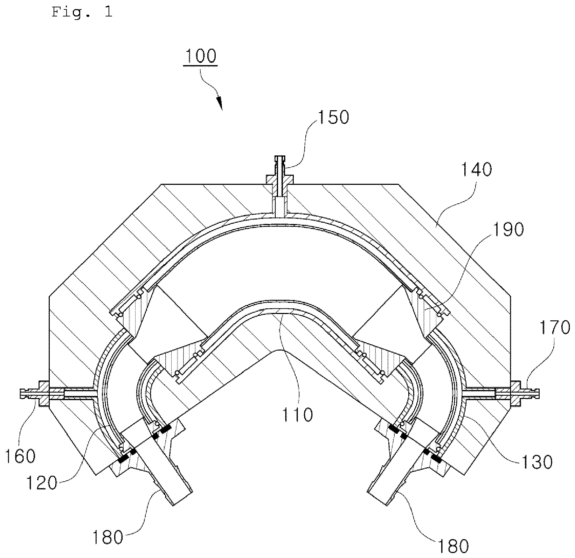

FIG. 1 is a cross-sectional view of the entire construction of a blood pump 100 according to an embodiment of the present disclosure.

Referring to FIG. 1, the blood pump 100 according to an embodiment of the present disclosure may be constructed to include a blood chamber 110, an inlet valve 120 for a pump, and an outlet valve 130 for a pump.

In this case, the inlet valve 120 for a pump is coupled to one end of the blood chamber 110 in a way that a fluid communicates therewith, and blocks a flow of blood into the blood chamber 110.

The outlet valve 130 for a pump is coupled to the other end of the blood chamber 110 in a way that a fluid communicates therewith, and blocks a flow of blood from the blood chamber 110.

According to an embodiment, the blood pump 100 may be constructed to further include a case 140 in which the blood chamber 110, the inlet valve 120 for a pump, and the outlet valve 130 for a pump are accommodated. The case is for preventing movements of the blood chamber 110, the inlet valve 120 for a pump, and the outlet valve 130 for a pump, which may occur as gas is injected into the blood chamber 110, the inlet valve 120 for a pump, and the outlet valve 130 for a pump. However, the case 140 is not an essential component of the present disclosure, and an implementation of the blood pump 100 according to an embodiment of the present disclosure is not impossible although the component of the case 140 is omitted.

In this case, the case 140 may be equipped with injection ports 150, 160, 170, and 180 through which a fluid communicates with the blood chamber 110, the inlet valve 120 for a pump, or the outlet valve 130 for a pump.

The case 140 is not limited to a shape of the case illustrated in FIG. 1, and may be modified in a shape in which the blood chamber 110, the inlet valve 120 for a pump, and the outlet valve 130 for a pump can be accommodated.

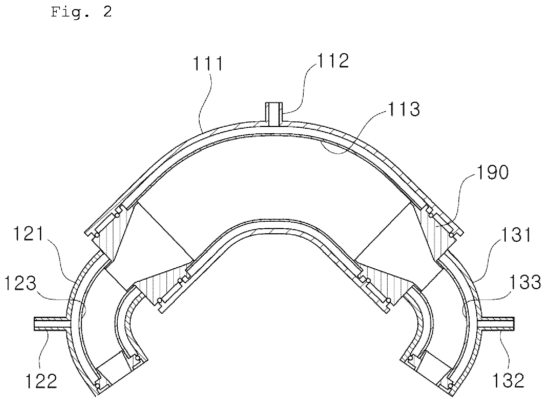

FIG. 2 is a diagram illustrating a structure and connection relation of components of the blood pump 100 according to an embodiment of the present disclosure.

Referring to FIG. 2, the blood chamber 110 of the blood pump 100 according to an embodiment of the present disclosure may be constructed to include an outer wall 111 having a gas inflow/outflow port 112 formed at one end thereof and a pouch 113 inserted into the outer wall 111 and having both ends coupled to both ends of the outer wall 111, respectively.

An outward form of each of the outer wall 111 and the pouch 113 may be fabricated in a bent pipe shape. In this case, it is preferred that the pouch 113 is disposed to be completely close to the outer wall 111.

The bent pipe means that curvature thereof is not extended or contracted if one side of the pouch 113 is deformed by pneumatic pressure and one side of the pouch 113 deformed by pneumatic pressure comes into contact with the other side of the pouch 113.

According to an embodiment, the inlet valve 120 for a pump or the outlet valve 130 for a pump may further include a valve outer wall 121, 131 having a gas inflow/outflow port 122, 132 formed at one end thereof and a valve pouch 123, 133 inserted into the valve outer wall 121, 131 and having both ends coupled to both ends of the valve outer wall 121, 131, respectively.

Furthermore, according to an embodiment, an outward form of each of the valve outer wall 121, 131 and the valve pouch 123, 133 may be fabricated in a bent pipe shape. In this case, curvature of the bent pipe means that the curvature is not extended or contracted if one side of the pouch 113 is deformed by pneumatic pressure and one side of the pouch 133 deformed by pneumatic pressure comes into contact with the other side of the pouch 133.

According to an embodiment, the blood pump 100 may be constructed to further include a connection unit 190. The connection unit 190 may further include a connection unit 190 having both ends coupled to the blood chamber 110, and the inlet valve 120 for a pump or the outlet valve 130 for a pump, respectively, and having an internal diameter that is increased from an end of the connection unit 190 coupled to the inlet valve 120 for a pump or the outlet valve 130 for a pump toward an end of the connection unit 190 coupled to the blood chamber 110.

Due to the addition of the component of the connection unit 190, a difference between the sizes of cross sections of the end of the blood chamber 110 and the ends of the inlet/outlet valves 120 and 130 for a pump can be reduced. A turbulence of blood, which may occur due to a difference between the sizes of cross sections of the ends of the components within the blood pump 100, can be reduced.

According to an embodiment, coupling between the components of the blood chamber 110, the inlet valve 120 for a pump, the outlet valve 130 for a pump, or the connection unit 190 may be embodied by a fitting method. In this case, a coupled portion coupled by the fitting method may be bonded by using an adhesive that has substantially the same material as a material that forms each of the components.

According to an embodiment, it is preferred that the blood pump 100 is fabricated to not have a gap, which may be formed by coupling between components, in a passage along which blood within the blood pump 100 flows, by coating the inside of the blood pump 100 by using the adhesive. This is for preventing a blood clotting foreign material, etc. which may be formed as blood is caught in the gap present within the passage.

FIG. 2 illustrates that the blood chamber 110 and the inlet/outlet valve 120, 130 for a pump are disposed on the same plane. However, it will be preferred that in order to reduce the total amount of blood necessary for the driving of the blood pump 100, an end that is not coupled to the blood chamber 110, among both ends of the inlet valve 120 for a pump and the outlet valve 130 for a pump, is coupled to the blood chamber 110 so that the end is directed toward a patient.

However, a method of coupling the blood chamber 110 and the inlet/outlet valve 120, 130 for a pump is not limited thereto. The design of the method may be properly changed within a range in which an operation of the blood pump 100 for forcing blood to flow in one direction may be performed.

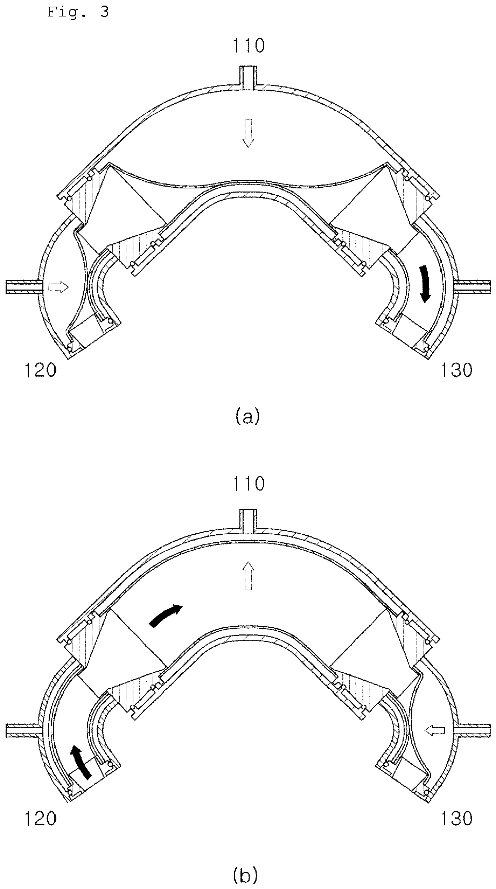

FIG. 3 is a diagram illustrating an operating process of the blood pump 100 according to an embodiment of the present disclosure. FIG. 3(a) illustrates a blood outflow process of the blood pump 100 according to an embodiment of the present disclosure, and FIG. 3(b) illustrates a blood inflow process thereof.

In FIG. 3, a white arrow indicates a movement of the pouch 113, and a black arrow indicates a flow of blood.

Referring to FIG. 3, the pouch 113 of the blood chamber 110 according to an embodiment of the present disclosure may have one side deformed by pneumatic pressure, thus forming a flow of blood. The pouch 113 may be formed in a hollow form having an elastic restoring force.

Furthermore, according to an embodiment, the valve pouch 123, 133 of the inlet valve 120 for a pump or the outlet valve 130 for a pump may have one side deformed by pneumatic pressure, thus blocking a flow of blood. The valve pouch 123, 133 may be formed in a hollow form having an elastic restoring force.

For example, as illustrated in FIG. 3(a), as one side of the valve pouch 123 of the inlet valve 120 for a pump is deformed by pneumatic pressure, a flow of blood from the blood chamber 110 to the direction of the inlet valve 120 for a pump may be blocked. As one side of the pouch 113 of the blood chamber 110 is deformed by pneumatic pressure, a flow of blood from the blood chamber 110 to the direction of the outlet valve 130 for a pump may be formed.

Furthermore, as illustrated in FIG. 3(b), as one side of the valve pouch 133 of the outlet valve 130 for a pump is deformed by pneumatic pressure, a flow of blood from the blood chamber 110 to the direction of the outlet valve 130 for a pump may be blocked. As one side of the pouch 113 of the blood chamber 110 is restored, a flow of blood from the inlet valve 120 for a pump to the direction of the blood chamber 110 may be formed.

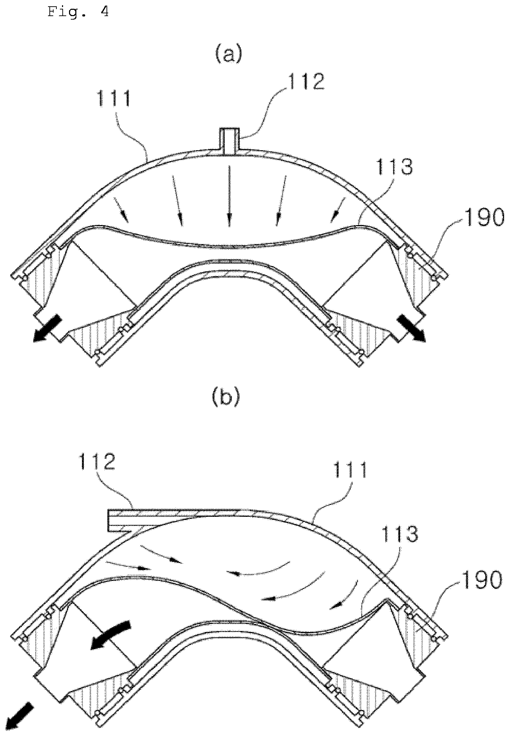

FIG. 4 is a diagram illustrating an operation of the blood pump 100 depending on a location of the gas inflow/outflow port 112 of the blood pump 100 according to an embodiment of the present disclosure. In FIG. 4, a thick arrow indicates a flow of blood, and a thin arrow indicates a flow of air.

Referring to FIG. 4, in the blood chamber 110 of the blood pump 100 according to an embodiment of the present disclosure, pneumatic pressure is formed between the outer wall 111 and the pouch 113 by gas that is introduced or discharged through the gas inflow/outflow port 112.

According to an embodiment, it will be preferred that a direction in which the gas inflow/outflow port 112 is disposed in the outer wall 111 of the blood chamber 110 is a direction in which the center line of the gas inflow/outflow port 112 is orthogonal to the center line of the blood chamber 110. The reason for this is that blood accommodated in the pouch 113 of the blood chamber 110 can be more efficiently ejected in a case in which the center line of the gas inflow/outflow port 112 is orthogonal to the center line of the blood chamber 110, compared to a case in which the center line of the gas inflow/outflow port 112 and the center line of the blood chamber 110 are identical with each other.

FIG. 4 (a) is a case in which the center line of the blood chamber 110 and the center line of the gas inflow/outflow port 112 are identical with each other. FIG. 4(b) is a case in which the center line of the blood chamber 110 and the center line of the gas inflow/outflow port 112 are orthogonal to each other.

As illustrated in FIG. 4(a), if the center line of the blood chamber 110 and the center line of the gas inflow/outflow port 112 are identical with each other, gas that is introduced through the gas inflow/outflow port 112 is spread to the left and right of the gas inflow/outflow port 112. At this time, as the central part of the pouch 113 of the blood chamber 110 earlier starts to be deformed, blood that is present near a portion into which a flow of blood has been blocked by the inlet/outlet valve 120, 130 for a pump remains within the pouch 113 without being ejected.

In contrast, as illustrated in FIG. 4(b), if the center line of the blood chamber 110 and the center line of the gas inflow/outflow port 112 are orthogonal to each other, gas that is introduced through the gas inflow/outflow port 112 is spread in a direction opposite to a direction in which the gas has been introduced. At this time, the pouch 113 of the blood chamber 110 is deformed in the direction opposite to the direction in which the gas has been introduced.

That is, in FIG. 4(b), since one end of the pouch 113 of the blood chamber 110 is deformed earlier than the central part thereof, the amount of blood that remains within the pouch 113 without being ejected is reduced unlike in FIG. 4(a).

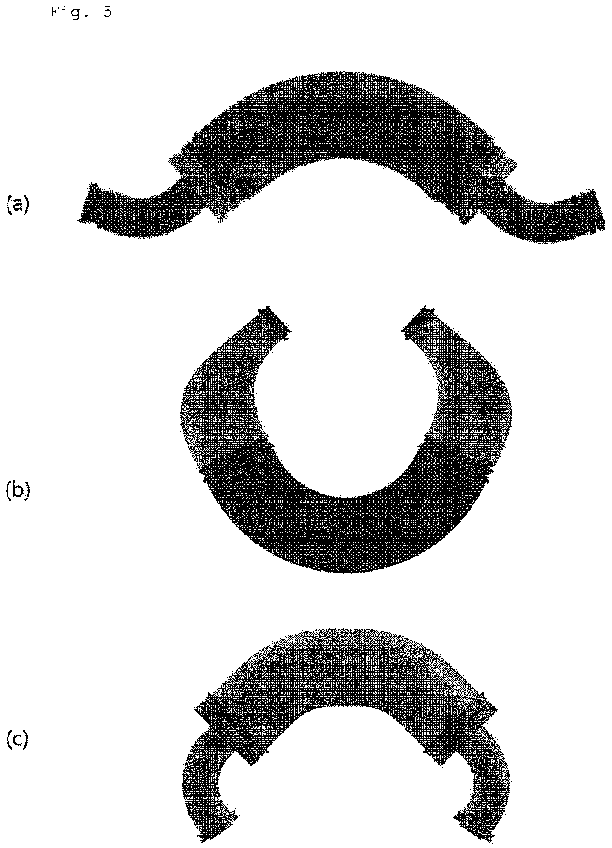

FIG. 5 illustrates various embodiments of the blood pump 100 according to the present disclosure, which are differently implemented depending on their purposes. Outward appearances of the blood pump 100 according to various embodiments of the present disclosure, which are illustrated in FIGS. 5(a) to 5(c), have different structures, but each of the structures is constructed to include the blood chamber 110, the inlet valve 120 for a pump, and the outlet valve 130 for a pump.

Referring to FIG. 5, in the blood pump 100 according to an embodiment of the present disclosure, as illustrated in FIG. 5(a), directions in which the inlet valve 120 for a pump and the outlet valve 130 for a pump are bent are implemented to be different from a direction in which the blood chamber 110 is bent. Accordingly, the size of the entire blood pump 100 can be reduced.

Furthermore, as illustrated in FIG. 5(b), as directions in which the inlet valve 120 for a pump and the outlet valve 130 for a pump are bent are implemented to be substantially the same as the direction in which the blood chamber 110 is bent, flows of blood within the inlet valve 120 for a pump, the blood pump 100, and the outlet valve 130 for a pump can be unified in one direction. As cross sections of the inlet valve 120 for a pump and the outlet valve 130 for a pump are implemented to be gradually increased toward the direction of the blood chamber 110, one ends of the inlet valve 120 for a pump and the outlet valve 130 for a pump have substantially the same cross section as both ends of the blood chamber 110. Accordingly, A turbulence of blood which may occur within the blood pump 100 can be reduced without adding the component of the connection unit 190 (i.e., without an additional component).

Furthermore, as illustrated in FIG. 5(c), as directions in which the inlet valve 120 for a pump and the outlet valve 130 for a pump are bent are implemented to be substantially the same as the direction in which the blood chamber 110 is bent, flows of blood within the blood pump 100 can be unified in one direction. As the sizes of cross sections of the inlet/outlet valves 120 and 130 for a pump are reduced, the volume of the blood pump 100 can be reduced, and the amount of blood necessary for the blood pump 100 can be reduced. Furthermore, as the component of the connection unit 190 is added between the blood chamber 110 and the inlet/outlet valves 120 and 130 for a pump, A turbulence of blood which may occur within the blood pump 100 may be reduced.

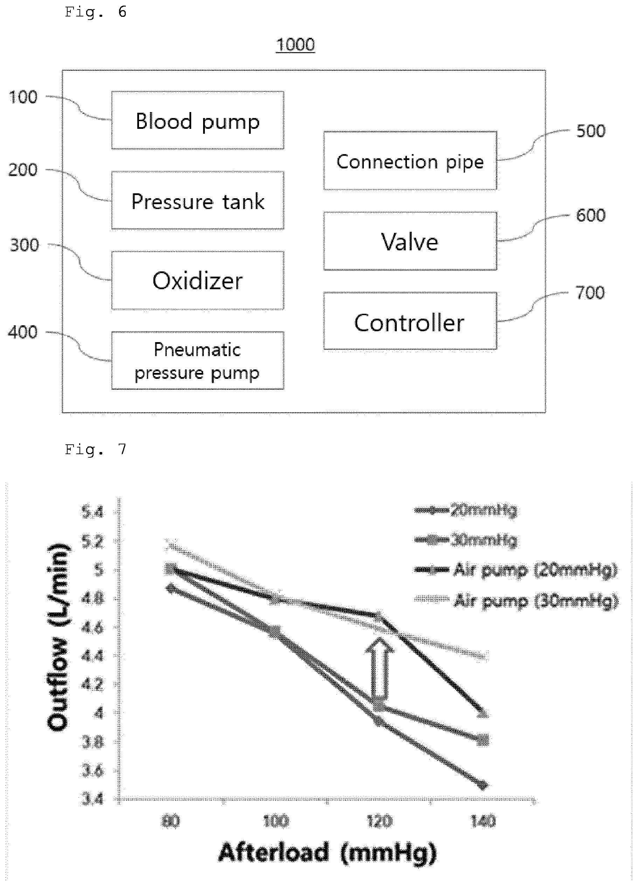

FIG. 6 is a block diagram of a blood oxygenation system 1000 according to an embodiment of the present disclosure.

Referring to FIG. 6, the blood oxygenation system 1000 according to an embodiment of the present disclosure may be constructed to include the aforementioned blood pump 100, a pressure tank 200 that accommodates compressed gas, an oxygenator 300 that oxidizes blood that is circulated by the blood pump 100, a connection pipe 500 that is a moving passage of gas and that connects the blood pump 100, the pressure tank 200, or the oxygenator 300, a plurality of valves 600 that is disposed in the connection pipe 500 and that controls a flow of gas that is introduced and discharged through the blood pump 100, and a controller 700 that controls an operation of the blood pump 100 by opening and closing the valves 600.

According to an embodiment, the blood oxygenation system 1000 may be constructed to further include a plurality of pressure sensors (not illustrated in the drawing).

According to an embodiment, the pressure sensors may be disposed in the blood chamber 110, the inlet valve 120 for a pump, and the outlet valve 130 for a pump, respectively.

Furthermore, according to an embodiment, the pressure sensor may be disposed in a 3-way solenoid valve to which a plurality of connection pipes 500 that connects the inlet valve 620 and the outlet valve 630 to the blood chamber 100 and the inlet valve 120 for a pump or the outlet valve 130 for a pump, respectively, is connected.

**At** least two pressure sensors may be disposed in each of the blood chamber 110, the inlet valve 120 for a pump, and the outlet valve 130 for a pump or may be disposed in three 3-way solenoid valves in order to ensure the accuracy of pressure measured by the pressure sensors.

In this case, the controller 700 may determine whether the valve 600 has failed based on pressure measured by the pressure sensor in a preset condition.

In this case, the preset condition means operating states or operating processes of the inlet valve 620 and the outlet valve 630.

For example, an operating state, such as whether the state of the inlet valve 620 or the outlet valve 630 is an opened state or whether both the inlet valve 620 and the outlet valve 630 are in a closed state, and an operating process, such as whether a valve that has been opened before is the outlet valve 630 or the inlet valve 620, may be set as the condition.

That is, if pressure of the inlet valve 620 and pressure of the outlet valve 630, which are measured by the pressure sensors, are 200 mmHg and 0 mmHg, respectively, when the inlet valve 620 and the outlet valve 630 operate normally, the controller 700 may determine that the inlet valve 620 or the outlet valve 630 has failed, when pressure of the inlet valve 620 measured by the pressure sensor is not 200 mmHg in the state in which the inlet valve 620 has been opened or when pressure of the outlet valve 630 measured by the pressure sensor is not 0 mmHg in the state in which the outlet valve 630 has been opened.

Furthermore, if both the inlet valve 620 and the outlet valve 630 are in the closed state and a value that has been opened before is the outlet valve 630, the controller 700 may determine that the inlet valve 620 has failed when pressure measured by the pressure sensor is close to 200 mmHg. If both the inlet valve 620 and the outlet valve 630 are in the closed state and a value that has been opened before is the inlet valve 620, the controller 700 may determine that the outlet valve 630 has failed when pressure measured by the pressure sensor is close to 0 mmHg.

According to an embodiment, the blood oxygenation system 1000 may be constructed to include the plurality of blood pumps 100. In this case, the controller 700 may control the plurality of blood pumps 100 so that blood is discharged from some of the plurality of blood pumps 100 and blood is introduced into the remaining blood pumps 100. Accordingly, a flow of blood that is ejected from all of the blood pumps 100 may be controlled to be not stopped.

To eject blood accommodated in the pouch 113 of the blood pump 100 is an operation that may be performed within a relatively short time, whereas to introduce blood into the pouch 113 of the blood pump 100 corresponds to an operation that requires a relatively long time.

Accordingly, it will be preferred that a flow of blood ejected from all of the blood pumps 100 is not stopped by discharging blood from the remaining blood pumps 100 while blood is introduced into some of the blood pumps 100.

According to an embodiment, the plurality of blood pumps 100 may be connected in parallel. In this case, the plurality of blood pumps 100 may be disposed to overlap.

For example, to dispose the plurality of blood pumps 100 so that they overlaps may mean that the components of the blood chamber 110, inlet valve 120 for a pump, and outlet valve 130 for a pump of one blood pump 100 are disposed to correspond to the components of the blood chamber 110, inlet valve 120 for a pump, and outlet valve 130 for a pump of each of the remaining blood pumps 100, respectively.

In this case, it will be preferred that the plurality of injection ports communicating with components (i.e., the blood chamber 110, the inlet valve 120 for a pump, and the outlet valve 130 for a pump) included in the blood pump 100, respectively, in a way that a fluid communicates therewith is fabricated to have the same direction on the basis of the components of the blood pump to which the injection ports are connected, respectively.

Furthermore, according to an embodiment, each of the plurality of blood pumps 100 connected in parallel may be constructed to further include a fixing unit having both sides correspond to one side of the blood pump 100, having a bottom correspond to the bottom of the blood pump 100, and having a partition plate for distinguishing between the blood pumps 100.

However, a shape in which the plurality of blood pumps 100 is connected and disposed in parallel is not limited thereto, and may be deformed in various shapes if the shape may have an effect obtained when the plurality of blood pumps 100 is connected in parallel.

FIG. 7 is a graph illustrating the amounts of blood that is ejected by the blood pump 100 before and after the component of a pneumatic pressure pump 400 is added in the blood oxygenation system 1000 according to an embodiment of the present disclosure.

According to an embodiment, the blood oxygenation system 1000 may be constructed to further include the pneumatic pressure pump. The pneumatic pressure pump is disposed in the connection pipe 500 that connects the blood pump 100 and the oxygenator 300, and forms vacuum in the blood pump 100 by sucking gas supplied to the blood pump 100.

In the graph of FIG. 7, a longitudinal axis means an afterload, and a transversal axis means the outflow of the blood pump 100 per minute. The afterload means the degree of tension that is formed by the ventricles of the heart in order to send blood from the left ventricle of the heart to the aorta during ventricular systole, and is also called a pressure load.

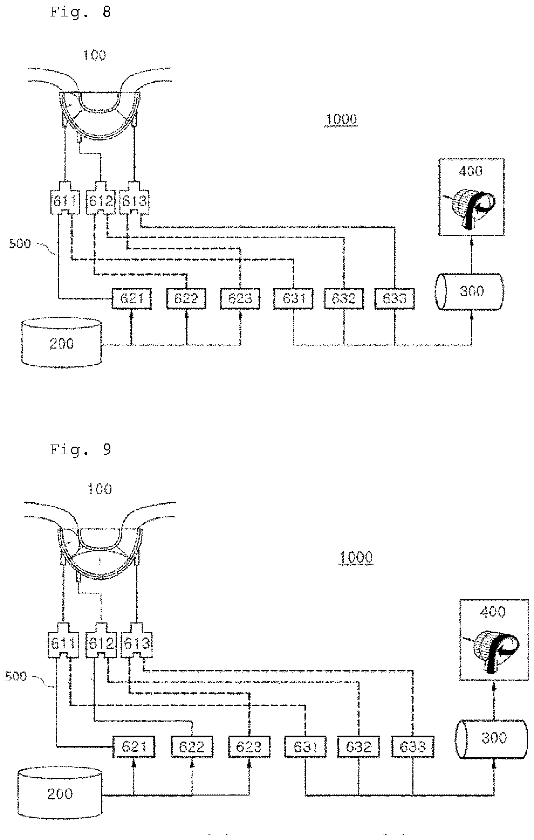

Referring to FIG. 8, it may be seen that the flow rate of the blood pump 100 can be further increased in some sections when the component of the pneumatic pressure pump is added.

That is, the pneumatic pressure pump 400 can improve a restoring force of the pouch 113 or the valve pouch 123, 133 by forming vacuum between the pouch 113 (or the valve pouch 123, 133) and the outer wall 111 (or the valve outer wall 121, 131) of the blood pump 100 in a way to suck gas supplied to the blood pump 100 by a pressure tank 200. Accordingly, the flow rate of the blood pump 100 can be increased by increasing the amount of blood that is introduced into the blood pump 100.

FIGS. 8 to 13 are diagrams illustrating an operation that is performed by the controller 700 of the blood oxygenation system 1000 according to an embodiment of the present disclosure. In FIGS. 8 to 13, a solid line means the connection pipe 500 in which a flow of gas is present, and a dotted line means the connection pipe 500 in which a flow of gas is not present.

Referring to FIG. 8, in the blood oxygenation system 1000 according to an embodiment of the present disclosure, gas may be oxygen. The oxygen may move from the pressure tank 200 to the blood pump 100 and from the blood pump 100 to the oxygenator 300 through the connection pipe 500.

That is, the gas that is supplied to the blood pump 100 and that is used to operate the blood pump 100 by forming pneumatic pressure is subsequently transferred to the oxygenator 300, and is used to oxidize blood that is supplied to the oxygenator 300 by the blood pump 100.

The valves 600 of the blood oxygenation system 1000 according to an embodiment of the present disclosure, which have been briefly illustrated in FIGS. 8 to 13, may be constructed to include a connection valve 610, an inlet valve 620, and a outlet valve 630.

In this case, the connection valve 610 may include a first connection valve 611 having one end connected to the inlet valve 120 for a pump, a second connection valve 612 having one end connected to the blood chamber 110, and a third connection valve 613 having one end connected to the outlet valve 130 for a pump.

Furthermore, the inlet valve 620 may include a first inlet valve 621 having both ends connected to the pressure tank 200 and the first connection valve 611, respectively, a second inlet valve 622 having both ends connected to the pressure tank 200 and the second connection valve 612, respectively, and a third inlet valve 623 having both ends connected to the pressure tank 200 and the third connection valve 613, respectively.

Furthermore, the outlet valve 630 may include a first outlet valve 631 having both ends connected to the oxygenator 300 and the first connection valve 611, respectively, a second outlet valve 632 having both ends connected to the oxygenator 300 and the second connection valve 612, respectively, and a third outlet valve 633 having both ends connected to the oxygenator 300 and the third connection valve 613, respectively.

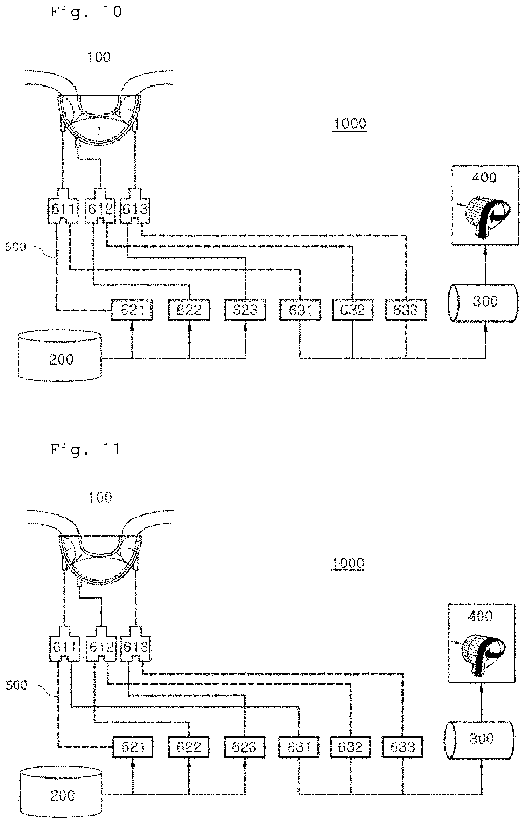

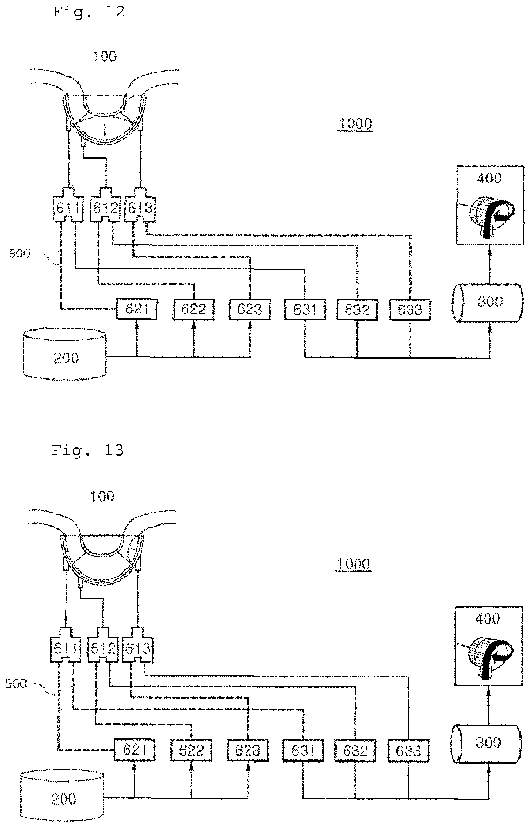

In this case, the controller 700 of the blood oxygenation system 1000 according to an embodiment of the present disclosure enables the blood chamber 110 to perform a series of operations. The operation may include a step (FIG. 8) of supplying the gas to the inlet valve 120 for a pump by opening the first inlet valve 621 in the state in which only the third outlet valve 633 has been opened, a step (FIG. 9) of supplying the gas to the blood chamber 110 by opening the second inlet valve 622 and closing the third outlet valve 633, a step (FIG. 10) of supplying the gas to the outlet valve 130 for a pump by closing the first inlet valve 621 and opening the third inlet valve 623, a step (FIG. 11) of sucking the gas from the inlet valve 120 for a pump by closing the second inlet valve 622 and opening the first outlet valve 631, a step (FIG. 12) of sucking the gas from the blood chamber 110 by closing the third inlet valve 623 and opening the second outlet valve 632, and a step (FIG. 13) of sucking the gas from the outlet valve 130 for a pump by closing the first outlet valve 631 and opening the third outlet valve 633.

According to an embodiment, the controller 700 enables the blood pump 100 to repeatedly perform the operation by controlling the opening and closing of the inlet valve 620 and the outlet valve 630.

In this case, the number of repetitions of the operation may be calculated on the basis of the heart rate of a patient. Furthermore, a ratio of the time that is taken for each of the steps of the operation may be calculated on the basis of a volume ratio that is occupied by each of the blood chamber 110, inlet valve 120 for a pump, and outlet valve 130 for a pump of the blood pump 100.

For example, if the heart rate (HR) of a patient is 60 bpm, the controller 700 may set the number of repetitions of the operation to 60 times per minute. In this case, a portion of the time for each of the steps included in the operation (i.e., a ratio of times taken for the steps FIGS. 8 to 13) may be calculated in proportion to a volume ratio of the blood chamber 110, inlet valve 120 for a pump, and outlet valve 130 for a pump of the blood pump 100.

In this case, the heart rate of the patient may be calculated based on a PPG signal or an ECG signal that is measured from a patient through a photoplethysmogram (PPG) sensor or an electrocardiogram (ECG) sensor, and may be a value that is input by a user (e.g., a doctor or a nurse) of the blood oxygenation system 1000.

The description of the present disclosure is illustrative, and a person having ordinary knowledge in the art to which the present disclosure pertains will understand that the present disclosure may be easily modified in other detailed forms without changing the technical spirit or essential characteristic of the present disclosure. Accordingly, it should be construed that the aforementioned embodiments are only illustrative in all aspects, and are not limitative. For example, elements described in the singular form may be carried out in a distributed form. Likewise, elements described in a distributed form may also be carried out in a combined form.

The scope of the present disclosure is defined by the appended claims rather than by the detailed description, and all changes or modifications derived from the meanings and scope of the claims and equivalents thereto should be interpreted as being included in the scope of the present disclosure.

## Claims

1. A blood pump forcing blood to flow in one direction, comprising:
a blood chamber comprising an outer wall configured to have a gas inflow/outflow port formed at one end thereof and a pouch inserted into the outer wall and configured to have both ends coupled to both ends of the outer wall, respectively;
an inlet valve for a pump coupled to one end of the blood chamber in a way that a fluid communicates therewith and configured to block a flow of blood to the blood chamber; and
an outlet valve for a pump coupled to the other end of the blood chamber in a way that a fluid communicates therewith and configured to block a flow of blood from the blood chamber.

2. The blood pump of claim 1, wherein in the blood chamber, pneumatic pressure is formed between the outer wall and the pouch by a gas that is introduced and discharged through the gas inflow/outflow port.

3. The blood pump of claim 1, wherein in the blood chamber, an outward form of each of the outer wall and the pouch is a bent pipe shape.

4. The blood pump of claim 1, wherein one side of the pouch is deformed by pneumatic pressure so that a flow of blood is formed.

5. The blood pump of claim 1, wherein the pouch is formed in a hollow form having an elastic restoring force.

6. The blood pump of claim 1, wherein the inlet valve for a pump or the outlet valve for a pump further comprises:
a valve outer wall having the gas inflow/outflow port formed at one end thereof; and
a valve pouch inserted into the valve outer wall and configured to have both ends coupled to both ends of the valve outer wall, respectively.

7. The blood pump of claim 6, wherein an outward form of each of the valve outer wall and the valve pouch is a bent pipe shape.

8. The blood pump of claim 6, wherein one side of the valve pouch is deformed by pneumatic pressure so that a flow of blood is blocked.

9. The blood pump of claim 6, wherein the valve pouch is formed in a hollow form having an elastic restoring force.

10. The blood pump of claim 1, further comprising a connection unit configured to have both ends coupled to the blood chamber and the inlet valve for a pump or the outlet valve for a pump, respectively, and to have an internal diameter that is increased from an end of the connection unit coupled to the inlet valve for a pump or the outlet valve for a pump toward an end of the connection unit coupled to the blood chamber.

11. A blood oxygenation system comprising:
the blood pump according to any one of claims 1 to 10;
a pressure tank configured to accommodate a compressed gas;
an oxygenator configured to oxidize blood that is circulated by the blood pump;
a connection pipe that is a moving passage of the gas and configured to connect the blood pump and the pressure tank or the oxygenator;
a plurality of valves disposed in the connection pipe and configured to control a flow of the gas that is introduced and discharged through the blood pump; and
a controller configured to control an operation of the blood pump by opening and closing the valves.

12. The blood oxygenation system of claim 11, wherein:
the blood oxygenation system comprises the plurality of blood pumps, and
the controller controls the plurality of blood pumps so that blood is discharged from some of the plurality of blood pumps and blood is introduced into remaining blood pumps of the plurality of blood pumps.

13. The blood oxygenation system of claim 11, further comprising a pneumatic pressure pump disposed in a connection pipe that connects the blood pump and the oxygenator and configured to form vacuum in the blood pump by sucking the gas supplied to the blood pump.

14. The blood oxygenation system of claim 11, wherein:
the gas is oxygen, and
the gas is moved from the pressure tank to the blood pump and from the blood pump to the oxygenator through the connection pipe.

15. The blood oxygenation system of claim 11, further comprising a plurality of pressure sensors disposed in the blood chamber, the inlet valve for a pump, and the outlet valve for a pump, respectively.

16. The blood oxygenation system of claim 15, wherein the controller determines whether the valve has failed based on pressure measured by the pressure sensor in a preset condition.

17. The blood oxygenation system of claim 11, wherein the valves comprise:
a connection valve comprising a first connection valve having one end connected to the inlet valve for a pump, a second connection valve having one end connected to the blood chamber, and a third connection valve having one end connected to the outlet valve for a pump;
an inlet valve comprising a first inlet valve having both ends connected to the pressure tank and the first connection valve, respectively, a second inlet valve having both ends connected to the pressure tank and the second connection valve, respectively, and a third inlet valve having both ends connected to the pressure tank and the third connection valve, respectively; and
an outlet valve comprising a first outlet valve having both ends connected to the oxygenator and the first connection valve, respectively, a second outlet valve having both ends connected to the oxygenator and the second connection valve, respectively, and a third outlet valve having both ends connected to the oxygenator and the third connection valve, respectively.

18. The blood oxygenation system of claim 17, wherein:
the controller enables the blood chamber to perform a series of operations, and
the operation comprises:
supplying the gas into the inlet valve for a pump by opening the first inlet valve in a state in which only the third outlet valve has been opened;
supplying the gas into the blood chamber by opening the second inlet valve and closing the third outlet valve;
supplying the gas into the outlet valve for a pump by closing the first inlet valve and opening the third inlet valve;
sucking the gas from the inlet valve for a pump by closing the second inlet valve and opening the first outlet valve;
sucking the gas from the blood chamber by closing the third inlet valve and opening the second outlet valve; and
sucking the gas from the outlet valve for a pump by closing the first outlet valve and opening the third outlet valve.

19. The blood oxygenation system of claim 18, wherein:
the controller enables the blood chamber to repeatedly perform the operation, and
the number of repetitions is calculated on the basis of a heart rate of a patient.
